## Europäisches Patentamt
## European Patent Office
## Office européen des brevets

(19)

(11) Publication number: **0 057 001**
**B1**

(12) # EUROPEAN PATENT SPECIFICATION

(45) Date of publication of patent specification: **08.04.87**

(51) Int. Cl.⁴: **A 61 M 5/16,** B 65 D 41/32, A 61 J 1/00

(21) Application number: **82100475.1**

(22) Date of filing: **23.01.82**

(54) **Cap connector.**

(30) Priority: **26.01.81 US 228417**

(43) Date of publication of application:
**04.08.82 Bulletin 82/31**

(45) Publication of the grant of the patent:
**08.04.87 Bulletin 87/15**

(84) Designated Contracting States:
**AT BE CH DE FR GB IT LI NL SE**

(56) References cited:
**US-A-3 509 879**
**US-A-4 177 149**
**US-A-4 228 835**

(73) Proprietor: **PALL CORPORATION**
**30 Sea Cliff Avenue**
**Glen Cove New York 11542 (US)**

(72) Inventor: **Rosenberg, David J.**
**12 Francis Court**
**Glen Cove New York (US)**

(74) Representative: **Gille, Christian, Dipl.-Ing. et al**
**Türk, Gille + Hrabal Patentanwälte Bruckner**
**Strasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

In many types of medical treatment, such as blood transfusions, intravenous feeding and the administration of medicinal solutions, it is necessary to introduce liquids into a patient, sometimes in rather large amounts. When this is done, it is quite important that absolutely no contaminants such as bacteria be permitted to pass into the patient with the liquid, because of the danger of injury or infection, with possibly fatal consequences. It is also necessary to exclude air, in order to prevent the development of an embolism in the patient.

The liquids themselves that are to be administered can easily be made sterile, and kept so before administration. The possibility of contamination arises, however, when the storage receptacle is to be connected with the administration set. The connection must be made under such conditions that no contaminants or bacteria can enter the system at the connection.

Liquid administration apparatus of this type, particularly intravenous liquid administration apparatus, normally require a filter, to ensure that undesirable or foreign contaminants in gaseous or particulate form not be administered. Such filters are connected in series between the reservoir or supply of liquid to be administered, and the administration set that passes the liquid into the patient. Many types of filter assemblies have been provided, but these normally are separate components that have to be put in fluid connection between the reservoir or supply and the patient, increasing the number of connections that have to be made, and that must be made reliably leak-tight and contamination-proof, preventing, for example, the entry of bacteria and other microorganisms from the surrounding environment into the system.

A typical filter assembly for connection with intravenous liquid administration apparatus is that described in US—A—4,177,149. That filter assembly comprises, in combination, a filter housing; a filter chamber in the housing; an inlet member and an outlet member in the housing in fluid flow communication with the filter chamber; a filter in the filter chamber disposed across the line of fluid flow through the chamber from the inlet member to the outlet member so that all through flow must pass through the filter, and dividing the chamber into two portions, one upstream and one downstream of the filter; a vent in the housing, in flow communication with the upstream portion of the filter chamber; and a liquid-impermeable gas-permeable porous member disposed across the line of flow through the vent, so that all vent flow must pass through the member, the member restricting such flow to gas to which it is permeable; the inlet member being shaped for attachment to a supply of intravenous liquid for intravenous administration; and the outlet member being shaped for attachment to an intravenous administration apparatus.

In the preferred embodiment, the inlet member is provided with a spike, for piercing a supply chamber made of plastic film and forming a liquid-tight seal therewith, and the outlet member is provided with a standard fitting, such as a spike socket, for attachment to an intravenous administration apparatus similarly equipped with a spike of other standard fitting that can enter the socket.

Another type of device that is frequently inserted in fluid flow connection with intravenous administration set is a gas purge device or gas eliminator. Devices of this type are provided, for example, by US—A—3,520,416, US—A—3,631,654 and US—A—3,523,408. All of these devices have both inlet and outlet connections that are normally open to the atmosphere, and that must be put in fluid flow connection with the intravenous liquid administration apparatus in a bacteria-tight and liquid-tight seal.

Such devices can be made sterile before installation in an administration system, but they must be kept so until installation. For this purpose, bacteria-tight and leak-tight caps can be used, but a cap must be removed before connection in the administration system, and this gives an opportunity for contamination during installation. It would be desirable not to have to remove the cap to make the connection.

A known connector for bottles or plastic bags is closed at its one end with a break-off piece demarked by an annular weak area from the remainder of the connector, wherein the break-off piece contains a dead-end bore into which a pin of an insert held within the connector is inserted in order to hold the break-off piece at a short distance from the outlet end of the connector after said piece has been broken off (DE—A—2 716 783). Such a connector is not acceptable for the introduction of liquids into a patient because the broken off break-off piece of the connector remains in connection with the connector and is therefor held within the flowing liquid which is thus contaminated by bacteria and the like on the outer surface of the break-off piece. Moreover, the hollow break-off piece can be broken off in a satisfactory manner only because the inserted pin provides necessary rigidity.

In accordance with the present invention, a dual functional device is provided, capable of serving both as a cap, to protect an open inlet and/or outlet port of a fluid receptacle, a fluid supply reservoir or a filter assembly, so as to maintain the same sterile, and also of being easily converted into a connector that can be made to connect the receptacle or reservoir or filter assembly in a leak-tight and bacteria-tight seal into an administration system.

The cap connector in accordance with the invention is capable as a cap of closing off in a leak-tight bacteria-tight seal a delivery port of a receptacle carrying pharmaceutically-acceptable material, or a receiving port of a receiver receiving pharmaceutically acceptable material, or an inlet or outlet port of a filter assembly, and as a connector for making a leak-tight and bacteria-tight connection between any two of the delivery port of the receptacle, the receiving port of the receiver, and the inlet or outlet of the filter assembly, for passage of pharmaceutically-acceptable material therebetween, said cap connector comprising:

a) a housing of resilient plastic material;

b) the housing defining the walls of a through passage having one end adapted for attachment to one of the ports and another end adapted for attachment to another of the ports, the passage being adapted for carrying pharmaceutically-acceptable material therebetween;

c) the portions of the housing defining the passage walls at the said ends of the passage resiliently forming a bacteria-tight and leak-tight seal in a press-fit to the said ports;

d) a compact, not hollow cap closure closing off one end of the through passage, integral with the housing and of the same plastic material, demarked by an annular weak area from the remainder of the housing;

e) the other end of the through passage being open for attachment to the delivery port of the receptacle or the receiving port of a receiver;

f) the cap closure being removable by tearing it away from the housing at the weak area, to open that end of the passage and convert the cap into a connector, which can be attached at one end in a bacteria-tight and leak-tight seal to one of the said ports and at the other end in a bacteria-tight and leak-tight seal to another of the said ports and pharmaceutically acceptable material transferred therebetween via the through passage.

This cap connector can for example be used as a cover and protector for the outlet port downstream of the filter of a filter assembly according to US—A—4,177,149, as well as a cap for protection of the contents of a fluid supply reservoir or a fluid supply receptacle.

A preferred embodiment of the cap connector of the invention is illustrated in the drawings in which:

Figure 1 represents a longitudinal section through a cap connector of the invention;

Figure 2 is a detailed view of the cap portion of the cap connector, showing the frangible link between the closure and the remainder of the cap connector body or housing;

Figure 3 is an end view of the cap connector of Figure 1, viewed from the closure end; and

Figure 4 is a view showing the cap connector of the invention attached as a protector over the outlet connection of a filter assembly of US—A—4,177,149.

The housing has a resilient construction. A rigid construction, using rigid sheets or molded or cast plastic parts or tubing, or parts or tubing made of metal, makes it difficult for the cap connector to form a leak-tight and bacteria-tight seal with the other components of the system.

The housing can be transparent, in which case the functioning of the system can be observed without dismantling the cap connector.

It will be evident from the above that the housing can be constructed of resilient plastic material that is also transparent, such as polyethylene, polypropylene, polymethyl pentene-1, polyvinyl chloride, and vinyl chloride-vinylidene chloride copolymers. Translucent resilient materials such as polypropylene, and polyethylene can also be employed. Other plastic materials that are suitable include polyamides, polyesters, polyvinyl butyral, cellulose acetate, cellulose acetate-propionate, ethyl cellulose, and polyoxymethylene resins.

The housing material should of course be inert to the fluids being processed.

The housing can be formed in the desired configuration by casting or molding, such as by extrusion or injection molding. The frangible link between the closure portion and the remainder of the housing can also be formed by casting or molding. For simplicity of construction, the housing is best formed in one piece, but it is also possible to form the housing in two pieces, the closure forming one piece, and being bonded integrally to the remainder by solvent bonding or by heat-fusing or spin welding.

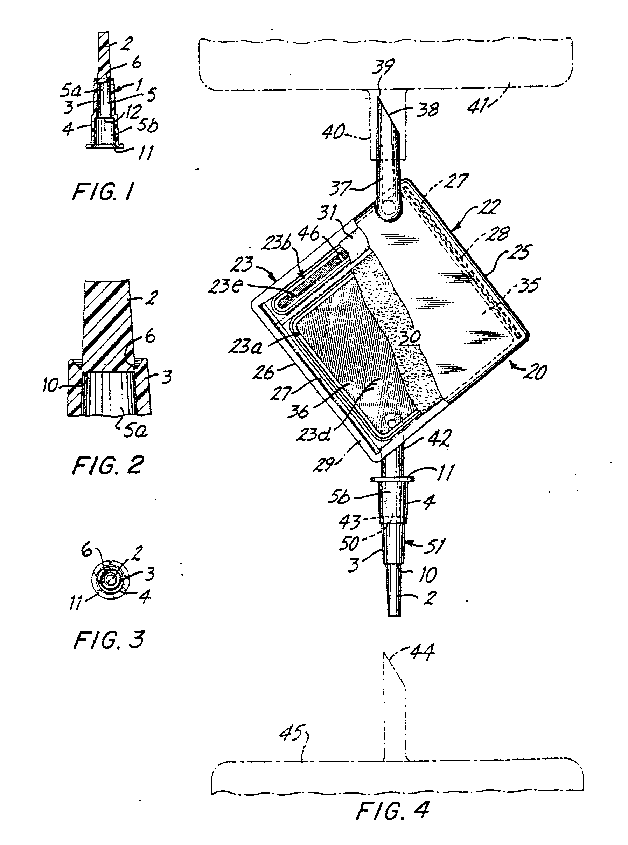

The cap connector shown in Figures 1 to 3 has a housing 1 of transparent resilient plastic material, such as polyvinyl chloride, polyvinylidene chloride; or of translucent material such as polypropylene or polyethylene or polyamide; or of opaque material such as acrylonitrile-butadienestyrene terpolymer, or a pigmented transparent or translucent material. The housing 1 is tubular, and shaped in three distinct, prominent portions, the closure or cap portion 2, an intermediate portion 3, and an end portion 4, with a central passage 5 therethrough, in two sections 5a, 5b of differing diameter, and tapered in the same directions. The closure portion 2 is integral with the remainder of the housing, but, as best seen in Figure 2, is linked thereto only at the thin annular connection 6. The connection portion is so thin and weak that it is quite easily broken off by simply pulling or tearing the closure off the remainder of the housing.

The closure closes off the outlet end 10 of the passage portion 5a. The passage 5a has a slight taper of $1\frac{1}{2}°$, the passage being smallest at the outlet end 10, but is essentially of uniform diameter.

At its opposite outlet end 11, the passage 5b is also tapered $1\frac{1}{2}°$, this time being wider at the open end 11 than at the inner end 12, where the passage 5b meets the passage 5a.

The passage 5b at the open end 11 is shaped to fit over the outlet connection 42 of a filter assembly of US—A—4,177,149, as seen in Figure 4, but it can also be shaped to receive, for example, the delivery port connector of a fluid supply container or reservoir, or the inlet port connector of a fluid receptacle. The configuration can readily be adapted as a female Luer socket to receive a standard male or fit into a standard female Luer fitting of such a connection from a standard supply reservoir or receptacle.

Similarly, at the other end 10 of passage 5a, the housing can have its exterior shaped as a male Luer fitting as to be received within a female Luer socket of a fluid supply receptacle, or of an administration set, or the inlet connection of a filter assembly, such as that of US—A—4,177,149. The outer surface of the housing is also tapered $1\frac{1}{2}°$, making the tip end slightly conical. This slight taper facilitates insertion over or removal from a fitting or port to which it is connected, and the resiliency of the material ensures a leak-tight and bacteria-tight seal therewith. Thus, in the case of the passage 5a and the housing at that end, and the passage 5b, this ensures a leak-tight and bacteria-tight connection with the supply reservoir, receptacle, or filter assembly port connection.

The utilization of the cap connector of *Figures 1 to 3* in this manner is illustrated in *Figure 4*.

The filter assembly shown in *Figure 4*, which is not object of the present invention, has a housing 20 of transparent rigid or semirigid plastic material, such as polyvinyl chloride, polymethyl acrylate, polymethyl methacrylate, or polyvinylidene chloride, or of translucent material, such as polypropylene, polyethylene, or polyamide, or opaque, such as acrylonitrile-butadiene-styrene terpolymer, polystyrene, or polycarbonate. The housing is a flattened cube, in two portions 22, 23. Each housing portion 22, 23 is shallowly dished with outer peripheral flanges 25, 26. Portion 23 has peripheral slots 27 on two sides and portion 22 has peripheral ribs 28 extending into the slots 27. Portion 22 also has ribs on the flanges 25, which are sacrificially integrated and thus bonded to housing portion 23 at flange 26 to form the completed housing such as, for example, by ultrasonic welding or by use of an adhesive or mutual solvent; these ribs are accordingly not shown.

The slots 27 are deep enough to receive the end portion 29 of the filter sheet 30, and permit the flanges 25, 26 to be integrated together without interference by the edge 29.

The edge 29 of the filter sheet 30 is held in a fluid-tight seal to the portion 23 on the outer side of slots 27, and the filter sheet extends from end to end and side to side of the major part 23a of portion 23. The filter sheet 30 is liquid-wetted, and can for example be a microporous hydrophilic nylon membrane. Beside the sheet 30 and in the same plane, but extending only over part 23b of portion 23, is a liquid-repellent-gas-permeable sheet 31, such as an expanded microporous polytetrafluoroethylene sheet. This sheet is also bonded to the portion 23 at its edges, in recess 23c.

The housing part 23a is ribbed, the ribs 23d extending diagonally across that part, while the part 23b is also ribbed, the ribs 23e extending parallel to the long sides of part 23b.

The housing encloses a filter chamber and since the entire peripheries of the sheets 30, 31 are sealed to the housing portion 23, the filter 30 and sheet 31 accordingly divide the filter chamber into two portions, an upstream portion 35, and a downstream portion 36.

Opening into the upstream portion 35 is an inlet member 37 of generally tubular configuration, terminating in a spike 38 with a sharp tip 39 for penetration of the fitting 40 (shown in dashed lines) of a plastic reservoir or storage vessel 41 containing liquid for intravenous administration, and forming a leak-tight seal therewith when penetrated into the vessel.

The downstream portion 36 of the filter chamber is provided with an outlet member 42, also generally of tubular configuration, and terminating in a tip 43.

The open end 50 of the outlet connector 42 is closed off by a protective cap 51 of the invention, the tip end entering the passage 5b at end 11 and sealing against the walls thereof in a fluid-tight and bacteria-tight seal.

When the closure tip 2 is removed, the connector passage 5a receives the corresponding spike 44 of an intravenous liquid administration kit 45, shown in dashed lines. Thus, liquid entering through the inlet member 37 must pass through the filter 30 in order to reach the outlet member 42 and the administration kit 45 via the connector 1.

Penetrating through the wall of the housing portion 23 and opening into part 23b is a vent 46, closed off by liquid-repellent gas-permeable filter sheet 31. Since this sheet is not wetted by the intravenous liquid being administered, since this is an aqueous fluid which wets hydrophilic materials but not hydrophobic materials, the pores of the polytetrafluoroethylene sheet are not penetrated by liquid, and therefore remain open for passage of gas therethrough at all times, which can accordingly escape via vent 46 from the housing.

In contrast, the pores of the filter 30 are saturated with liquid, immediately that liquid fills the upstream chamber 35, with the result that under gravity flow administration, as shown, the filter 30 is not penetrated by gas, but blocks the passage of gas therethrough. As a result, any gas entering with the liquid through the inlet cannot pass through the filter 30, but since it can pass through the sheet 31, it escapes through the vent 46.

It will be noted that the spike 38 and socket 43 are at opposite corners of the housing 21. This means that the device when installed assumes a position in which the sheet 31 extends diagonally upward, and so is in a position to vent via vent 46 all gas rising to the top of chamber 35, aided by the ribs 23e. The ribs 23d are thus vertical, aiding in guiding liquid to outlet 42.

Thus, in operation after the spike 38 of the filter assembly has been pushed into the female Luer fitting 55 of the liquid supply vessel as illustrated, liquid flows freely into the upstream chamber 35, passes through filter 30, and then enters the downstream chamber 36, whence it passes along the ribs 23d and leaves through the outlet member 42. The cap 51 is opened by snapping off the closure 2, thus opening the passage 5, and the open end 10 then receives the tip 44 of the intravenous administration kit 45, whence it is administered. Gas blocked from pass through the filter 30 escapes through the vent 46 via the liquid-repellent sheet 31.

Claims

1. A cap connector (51), capable as a cap of closing off in a leak-tight bacteria-tight seal, a delivery port of a receptacle carrying pharmaceutically-acceptable material, or a receiving port of a receiver receiving pharmaceutically-acceptable material, or an inlet or outlet port (37, 42) of a filter assembly (20) and as a connector for making a leak-tight and bacteria-tight connection between any two of said ports, namely the delivery port of the receptacle, the receiving port of a receiver, and the inlet or outlet port of the filter assembly, for passage of pharmaceutically-acceptable material therebetween, said cap connector comprising:

a) a housing (1) of resilient plastic material;

b) the housing (1) defining the walls of a through passage (5) having one end (11) adapted for attachment to one of the ports (42) and another end (10) adapted for attachment to another of the ports, the passage being adapted for carrying pharmaceutically-acceptable material therebetween;

c) the portions of the housing (1) defining the passage walls at the said ends (10, 11) of the passage (5) resiliently forming a bacteria-tight and leak-tight seal in a press-fit to the said ports (37, 42);

d) a compact, not hollow cap closure (2) closing off one end (10) of the through passage (5), integral with the housing (1) and of the same plastic material, demarked by an annular weak (6) area from the remainder of the housing;

e) the other end (11) of the through passage (5) being open for attachment to the delivery port (42) of the receptacle (20) or the receiving port (37) of a receiver;

f) the cap closure (2) being removable by tearing it away from the housing (1) at the weak area (6), to open that end of the passage (5) and convert the cap into a connector, which can be attached at one end in a bacteria-tight and leak-tight seal to one of the said ports (37 or 42) and at the other (10) end in a bacteria-tight and leak-tight seal to another of the said ports so that pharmaceutically-acceptable material can be transferred therebetween via the through passage.

2. A cap connector (51) according to claim 1, wherein the housing (1) is of polyvinyl chloride.

3. A cap connector (51) according to claim 1, wherein the housing (1) and associated parts (2) are made of transparent plastic.

4. A cap connector (51) according to claim 1, wherein the housing (1) and any other plastic parts (2) are solvent-bonded or fused together in a one-piece construction.

5. A filter (20) having an outlet member (42) and a cap connector (51) according to claim 1 capping the outlet member in a leak-tight and bacteria-tight seal.

Revendications

1. Connecteur formant capuchon (51) capable, sous forme d'un capuchon, de fermer avec un joint étanche aux fuites et aux bactéries, un orifice de distribution d'un récipient contenant une matière acceptable pharmaceutiquement ou un orifice de réception d'un organe récepteur destiné à recevoir une matière acceptable pharmaceutiquement, ou un orifice d'entrée ou de sortie (37, 42) d'un ensemble de filtrage (20), et, sous forme d'un connecteur, de former une connexion étanche aux fuites et aux bactéries entre deux quelconque des orifices précités, c'est-à-dire l'orifie de distribution de récipient, l'orifice de réception d'un récepteur, et l'orifice d'entrée ou de sortie de l'ensemble de filtration, afin qu'une matière acceptable pharmaceutiquement puisse circuler entre eux, le connecteur formant capuchon comprenant:

a) un boîtier (1) formé d'une matière plastique élastique,

b) le boîtier (1) délimitant les parois d'un passage (5) qui débouche et qui a une première extrémité (11) destinée à être fixée à l'un des orifices (42) et une autre extrémité (10) destinée à être fixée à un autre des orifices, le passage étant destiné à transmettre la matière acceptable pharmaceutiquement entre les orifices,

c) les parties du boîtier (1) délimitant les parois du passage auxdites extrémités (10, 11) du passage (5) formant élastiquement un joint étanche aux bactéries et aux fuites, en étant emmanchées à force sur les orifices (37, 42),

d) un organe (2) de fermeture de capuchon, de forme compacte mais non creux, fermant une première extrémité (10) du passage (5), solidaire du boîtier (1) et formé de la même matière plastique que celui-ci, est délimité par une zone annulaire de faiblesse (6) du reste du boîtier,

e) l'autre extrémité (11) du passage (5) étant ouverte afin qu'elle soit fixée à l'orifice de distribution (42) du récipient (20) ou à l'orifice de réception (37) d'un récepteur,

f) l'organe (2) de fermeture formant capuchon pouvant être retiré par arrachement du boîtier (1) dans la zone de faiblesse (6) de façon que l'extrémité correspondante du passage (5) soit ouverte et que le capuchon soit transformé en connecteur qui peut être fixé à une première extrémité, par un joint étanche aux bactéries et aux fuites, à l'un des orifices (37 ou 42), et à l'autre extrémité (10), par un joint étanche aux bactéries et aux fuites, à un autre des orifices afin qu'une matière acceptable pharmaceutiquement puisse être transférée entre les orifices par le passage transversal.

2. Connecteur (51) formant capuchon selon la revendication 1, dans lequel le boîtier (1) est formé de chlorure de polyvinyle.

3. Connecteur (51) formant capuchon selon la revendication 1, dans lequel le boîtier (1) et les parties associées (2) sont formés d'une matière plastique transparente.

4. Connecteur (51) formant capuchon selon la revendication 1, dans lequel le boîtier (1) et l'une quelconque des autres parties de matière plastique (2) sont liés par un solvant ou associés par fusion sous forme d'une construction en une seule pièce.

5. Filtre (20) ayant un organe de sortie (42) et un connecteur (51) formant capuchon selon la revendication 1, recouvrant l'organe de sortie en formant un joint étanche au fluide et aux bactéries.

**Patentansprüche**

1. Eine Verbindungskapsel (51) geeignet als Kapsel für eine leckdichte und bakteriendichte Abdichtung einer Auslassöffnung eines eine pharmazeutisch akzeptierbare Substanz enthaltenden Behälters oder einer Aufnahmeöffnung eines Empfängers einer pharmazeutisch akzeptierbaren Substanz, oder einer Einlass- oder Auslassöffnung (37, 42) einer Filteranordnung (20), und geeignet als Anschluss für die Herstellung einer leckdichten und bakteriendichten Verbindung zwischen zwei beliebigen Öffnungen, insbesondere der Abgabeöffnung des Behälters, der Aufnahmeöffnung eines Empfängers und der Einlass- oder Auslassöffnung der Filteranordnung, um dazwischen eine pharmazeutisch akzeptierbare Substanz zu befördern, und die folgendes aufweist:

a) ein Gehäuse (1) aus elastischem Plastikmaterial;

b) wobei das Gehäuse (1) die Wände eines Durchgangs (5) bildet, dessen eines Ende (11) dafür ausgelegt ist, um an einer der Öffnungen (42) befestigt zu werden, während das andere Ende (10) dafür ausgelegt ist, um an einer der anderen Öffnungen befestigt zu werden, und der Durchgang dafür ausgelegt ist, eine pharmazeutisch akzeptierbare Substanz dazwischen zu befördern;

c) wobei die die Wände des Durchgangs bildenden Teile des Gehäuses (1) an den Enden (10, 11) des Durchgangs (5) eine elastische bakteriendichte und leckdichte Abdichtung in einem Paßsitz in den Öffnungen (37, 42) bilden;

d) einen kompakten nicht hohlen Verschluss (2), der ein Ende (10) des Durchgangs (5) verschliesst, der integral mit dem Gehäuse (1) aus dem gleichen Plastikmaterial gebildet ist und durch einen ringförmigen Schwachbereich (6) vom restlichen Gehäuse abgegrenzt ist;

e) wobei das andere Ende (11) des Durchgangs (5) offen ist, um an die Abgabeöffnung (42) des Behälters (20) oder die Aufnahmeöffnung (37) eines Empfängers angeschlossen zu werden;

f) und die Verschlusskapsel (2) vom Gehäuse (1) durch Abbrechen am Schwachbereich (6) entfernt werden kann, um dieses Ende des Durchgangs (5) zu öffnen und die Kapsel in einen Anschluss zu verwandeln, der an einem Ende in bakteriendichter und leckdichter Abdichtung an einer der Öffnungen (37, 42) befestigt werden kann, während das andere Ende (10) in bakteriendichter und leckdichter Abdichtung an einer der anderen Öffnungen befestigt ist, so dass eine pharmazeutisch akzeptable Substanz über den Durchgang zwischen diesen öffnungen transportiert werden kann.

2. Eine Verbindungskapsel (51) nach Anspruch 1, in der das Gehäuse (1) aus Polyvinylchlorid hergestellt ist.

3. Eine Verbindungskapsel (51) nach Anspruch 1, in der das Gehäuse (1) und die zugehörigen Teile (2) aus transparentem Plastik hergestellt sind:

4. Eine Verbindungskapsel (51) nach Anspruch 1, in der das Gehäuse (1) und alle anderen Plastikteile (2) in einer einstückigen Konstruktion miteinander verklebt oder verschweisst sind.

5. Ein Filter (20) mit einem Auslass (42) und einer Verbindungskapsel (51) nach Anspruch 1, welche den Auslass in leckdichter und bakteriendichter Abdichtung abdeckt.

FIG. 1

FIG. 2

FIG. 3

FIG. 4